# EUROPÄISCHE PATENTSCHRIFT

(11) **EP 0 770 838 B1**
(45) Veröffentlichungstag und Bekanntmachung des Hinweises auf die Patenterteilung: **02.01.2002**
(21) Anmeldenummer: 96114771.7
(22) Anmeldetag: 14.09.1996
(51) Int. Cl.: F25C 1/22, F25D 25/00

(54) **Vorrichtung zur Formgebung von Beuteln**
Arrangement for shaping pouches
Arrangement pour façonner des sacs

(30) Priorität: 26.10.1995 DE 19539749
(43) Veröffentlichungstag der Anmeldung: 02.05.1997
(73) Patentinhaber: MESSER GRIESHEIM GMBH, 60547 Frankfurt (DE)
(72) Erfinder: Biederbick, Manfred, 47802 Krefeld (DE)

(56) Entgegenhaltungen:
- WO-A-90/05495
- WO-A-94/05247
- WO-A-95/09597
- US-A- 2 736 277
- US-A- 3 776 411

## Beschreibung

Die Erfindung betrifft eine Vorrichtung zur Formgebung von Beuteln, die mit Füllgut beschickt werden, welches eingefroren und gelagert werden soll.

Im medizinischen Bereich werden Stammzellenkonserven kryokonserviert. Hierzu wird die Stammzellenprobe in Beutel gefüllt, in Formgebungsvorrichtungen eingelegt und in diesen Formgebungsvorrichtungen eingefroren. Hierbei wird es ermöglicht, die sich in dem Beutel befindliche Stammzellenkonserve in einer definierten Schichtdicke zu halten, welche für das Einfrieren optimal ist. Hierzu ist nach dem Stand der Technik eine Vorrichtung bekannt, welche in der US-PS 4,469,227 beschrieben ist. Sie besteht im wesentlichen aus zwei über ein Scharnier in Verbindung stehenden Platten, welche zusammenklappbar sind und einen Beutel aufnehmen können. Um ein Herausfallen des Beutels zu verhindern und die Formgebung zu fixieren, sind Mittel angebracht, welche die beiden über das Scharnier in Verbindung stehenden Platten, zwischen denen sich der Beutel befindet, nach dem Zusammenklappen fixieren. Die Beutel sind mit Stutzen ausgestattet, welche zur Aufnahme von Stammzellenproben dienen und sperrig ausgebildet sind. Um diese Stutzen aufzunehmen, sind in den Platten Mulden vorgesehen, die der Form der Stutzen angepaßt sind. Bei der Vorrichtung nach der US-PS 4,469,227 sind die mit Stammzellenkonserven gefüllten Beutel und somit die Stutzen komplett in die Formgebungsvorrichtung eingebettet und somit nicht mehr von außen zugänglich. Sie sind keiner weiteren Handhabung zugänglich. Die Originalbeschriftung der Beutel kann bei diesen Vorrichtungen ebenfalls nicht abgelesen werden, so daß es zu Verwechslungen kommen kann.

Vorrichtungen gemäß dem Oberbegriff des Anspruchs 1 sind z.B. aus der WO-A-90/05495 oder der US-A-2 736 277 bekannt.

Der Erfindung liegt die Aufgabe zugrunde, die Form der Vorrichtung zur Aufnahme von Blutplasmabeuteln zu verbessern.

Ausgehend von dem im Oberbegriff des Anspruchs 1 berücksichtigten Stand der Technik ist die Aufgabe erfindungsgemäß gelöst mit den im kennzeichnenden Teil des Anspruchs 1 angegebenen Merkmalen.

Mit der erfindungsgemäßen Vorrichtung ist es nunmehr möglich, Konserven von Stammzellen oder auch Blut unter Formgebung zu gefrieren und zu lagern, ohne sie dabei einer Zugänglichkeit für die Handhabbarkeit zu entziehen. Verwechslungsmöglichkeiten werden verhindert.

Vorteilhafte Weiterbildungen sind in den Unteransprüchen angegeben.

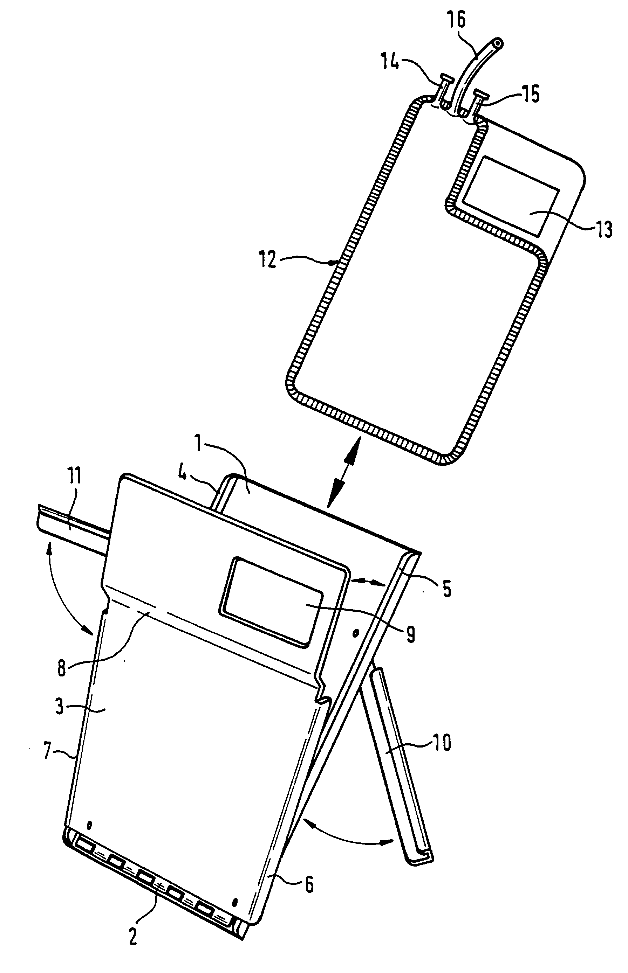

In der Zeichnung ist die erfindungsgemäße, kassettenförmig ausgebildete Vorrichtung mit einer Stammzellenkultur beispielhaft abgebildet.
In ihr ist eine Rückenplatte 1 über ein Scharnier 2 an eine Deckplatte 3 zusammenklappbar angebracht. Die Rückenplatte 1 und die Deckplatte 3 weisen Seitenkanten 4,5,6,7 auf, welche beim Zusammenklappen zur Ausbildung eines weitgehend geschlossenen Volumens führen.
Die Deckplatte 3 ist über eine Abkantung 8 derart ausgebildet, daß sie nach der Abkantung 8 in der dem Scharnier 2 entgegengesetzten Richtung in einem größeren Abstand zur Rückenplatte 1 parallel zur Rückenplatte 1 verläuft. In der Deckplatte 3 ist ein Fenster 9 angebracht. An der Rückenplatte 1 sind zur Fixierung der Deckplatte 3 Spannhalter 10 und 11 flexibel und unlösbar angebracht. In Einschubrichtung ist der Beutel 12 dargestellt, welcher ein Beschriftungsetikett 13 und Füllstutzen beziehungsweise Entnahmestutzen 14 und 15 sowie einen Schlauch 16 aufweist.

Soll nun eine Stammzellenkultur unter Formgebung eingefroren werden, indem sie beispielsweise in flüssigem Stickstoff auf tiefe Temperaturen abgekühlt werden, so legt man den Beutel 12, in welchem sich die Stammzellenkultur befindet, derart ein, daß die Stutzen 14 und 15 und der Schlauch 16 an der von dem Scharnier 2 abgewandten Seite zu liegen kommen und von dem Abschnitt der Deckplatte 3, der durch die Abkantung 8 in einem größeren Abstand zur Rückenplatte 1 gehalten wird, überdeckt wird. Durch Umlegen der Spannhalter 10 und 11 1 kann der Beutel 12 formgebend fixiert werden. Die gesamte Formgebungsvorrichtung wird nun mit dem Beutel 12 in das Gefriermittel gegeben und kann dort bis zum vollständigen Gefrieren oder darüber hinaus verweilen. Nach der erfindungsgemäßen Ausgestaltung der Vorrichtung kommen die operativen Einheiten des Beutels 12, wie der Entnahmestutzen 14, derart zu liegen, daß sie über eine Öffnung zugänglich sind, welche sich an der dem Scharnier 2 gegenüberliegenden Schmalseite befindet. Hierbei kann die Schmalseite ganz oder teilweise offen ausgebildet sein. Es ist auch möglich, die Vorrichtung derart auszubilden, daß die Öffnungen sich auch auf die Seitenkanten 4,5,6, und 7 der anderen Schmalseiten ausdehnen.

Die Arretierung der Rückenplatte 1 und der Deckplatte 3 kann alternativ zu den Spannhaltern 10 und 1 1 auch durch andere Mittel wie Klemmen oder Riegel erfolgen. Es ist auch möglich, anstelle der Deckplatte 3 die Rückenplatte 1 oder sowohl die Deckplatte 3 als auch die Rückenplatte 1 abzukanten um operative Bestandteile des Beutels 12, wie den Entnahmestutzen 14 zugänglich zu machen. Das Scharnier 2 kann auch seitlich angebracht sein, so daß die Vorrichtung seitlich aufgeklappt werden kann. Bei einer solchen Ausführungsform sind die Befestigungselemente am unteren Ende und an der anderen Seite angeordnet. In einer Abwandlung können als Formbegungsteile auch zwei lose Platten zusammengefügt werden, die mit Befestigungselementen zusammengehalten werden. In diesem Fall sind an den Innenseiten der Deckplatte 3 und der Rückenplatte 1 Mittel angebracht, mit denen der Beutel 12 gegen ein Verrutschen gesichert werden kann. Die Abmessungen der Formgebungsvorrichtung richten sich nach den jeweiligen Anforderungen an das zu gefrierende Volumen in Verbindung mit der angestrebten Schichtdicke. So sind beispielsweise Volumina von 100ml oder 20-40ml üblich. Die Formgebungsvorrichtung mit den Formgebungshälften ist für die Beuteltypen 4R5462, 4R5461, 4R9955 sowie FTX 1052 der Firma Baxter Deutschland GmbH und die Typen DF-700/2 und DF 200/2 der Firma Biotrans-Deutschland. Die Position des Fensters 9 zur Sichtbarmachung des Beschriftungsetikettes befindet sich je nach Beutelart an verschiedenen Stellen. Die Deckplatte 3 oder die Rückenplatte 1 kann etwas verkürzt sein, so daß die jeweils andere Seite übersteht. Somit ist über die offene Schmalseite ein noch leichterer Zugang zu den operativen Elementen der Beutel 12 wie Stutzen 14, 15, möglich. Die Ausgestaltung der Vorrichtung ist natürlich nicht auf die rechteckige Form beschränkt, vielmehr sind auch mehreckige oder abgerundete Formen denkbar.
Mit der erfindungsgemäßen Vorrichtung ist es nunmehr möglich, Beutel mit zu gefrierendem Inhalt formgebend zu gefrieren ohne dabei eine Zugänglichkeit der Probe einer weiteren Handhabung an den operativen Elementen zu entziehen. Verwechslungsfehler durch uneinheitliche Beschriftung von Beutel und Formgebungsvorrichtung können verhindert werden. Es können Stammzellenkulturen, Blut aber auch andere teilweise feste, weil inhomogene Stoffe in ihrer Formgebung fixiert werden.
Nach dem Gefrieren ist eine Lagerung der Konserven in tiefkaltem Kühlmittel, wie flüssigem Stickstoff oder kaltem Stickstoff in der Gasphase möglich, dabei werden die tiefkalten Beutel in der Vorrichtung vor mechanischer Beanstandung geschützt.

## Patentansprüche

1. Vorrichtung umfassend zwei plattenförmig ausgebildete Formgebungshälften (1, 3), welche zusammenklappbar miteinander verbunden sind, wobei die Vorrichtung in geschlossenem Zustand an einer Schmalseite eine Öffnung aufweist, **dadurch gekennzeichnet, daß** mindestens eine der Formgebungshälften (1, 3) durch eine Abkantung (8) derart in ihrem Verlauf abgebogen ist, daß sie bei geschlossener Vorrichtung in Richtung der offenen Schmalseite in einem größeren Abstand zur anderen Formgebungshälfte parallel verläuft.

2. Vorrichtung nach Anspruch 1, **dadurch gekennzeichnet, daß** die Formgebungshälften (1, 3) Seitenkanten (4, 5, 6, 7) aufweisen.

3. Vorrichtung nach Anspruch 2, **dadurch gekennzeichnet, daß** sich die Öffnung der Vorrichtung auf die Seitenkanten (4, 5, 6, 7) ausdehnt.

4. Vorrichtung einem der Ansprüche 1 bis 3, **dadurch gekennzeichnet, daß** an der als Rückenplatte dienenden Formgebungshälfte (1) Spannhalter (10, 11) flexibel und unlösbar angebracht sind.

5. Vorrichtung nach einem der Ansprüche 1 bis 4, **dadurch gekennzeichnet, daß** eine der Formgebungshälften (1) der Vorrichtung an der in geschlossenem Zustand geöffneten Schmalseite gegenüber der anderen Formgebungshälfte (3) verkürzt ist.

6. Vorrichtung nach einem der Ansprüche 1 bis 5, **dadurch gekennzeichnet, daß** die Vorrichtung ein Fenster (9) aufweist.

## Claims

1. Apparatus comprising two shaping halves (1, 3) which are of plate-like design and are connected to one another such that they can be swung together, the apparatus, in the closed state, having an opening on a narrow side, **characterized in that** at least one of the shaping halves (1, 3) is bent in profile by way of an angled section (8) such that, with the apparatus closed, it runs parallel, in the direction of the open narrow side, to the other shaping half, at a relatively large distance therefrom.

2. Apparatus according to Claim 1, **characterized in that** the shaping halves (1, 3) have side edges (4, 5, 6, 7).

3. Apparatus according to Claim 2, **characterized in that** the opening of the apparatus extends to the side edges (4, 5, 6, 7).

4. Apparatus according to one of Claims 1 to 3, **characterized in that** tensioning holders (10, 11) are fitted in a flexible and non-releasable manner on the shaping half (1), which serves as the rear plate.

5. Apparatus according to one of Claims 1 to 4, **characterized in that** one of the shaping halves (1) of the apparatus is shorter than the other shaping half (3) on the narrow side which is open in the closed state.

6. Apparatus according to one of Claims 1 to 5, **characterized in that** the apparatus has a window (9).

## Revendications

1. Arrangement comprenant deux demi éléments de façonnage (1, 3) en forme de plaques, reliés ensemble par une charnière, l'arrangement présentant, à l'état fermé, une ouverture sur son petit côté,
**caractérisé en ce qu'**
un au moins des deux demi-éléments de façonnage (1, 3) est recourbé dans sa longueur par un repli (8) de sorte que, en position fermée de l'arrangement, en direction du petit côté ouvert, il se trouve décentré parallèlement d'une distance accrue par rapport à l'autre demi-élément de façonnage.

2. Arrangement selon la revendication 1,
**caractérisé en ce que**
les demi-éléments de façonnage (1, 3) présentent des replis latéraux (4, 5, 6, 7).

3. Arrangement selon la revendication 2,
**caractérisé en ce que**
l'ouverture de l'arrangement s'étend aux replis (4, 5, 6, 7).

4. Arrangement selon l'une des revendications 1 à 3,
**caractérisé en ce que**
sur la première moitié (1) de l'élément de façonnage servant de dosseret, sont montées des barrettes d'écartement (10, 11) flexibles et indémontables.

5. Arrangement selon l'une des revendications 1 à 4,
**caractérisé en ce que**
le petit côté de l'un des demi-éléments (1) est raccourci en position fermée par rapport à celui de l'autre demi-élément (2)

6. Arrangement selon l'une des revendications 1 à 5,
**caractérisé en ce que**
l'arrangement comporte une fenêtre (9).
